Europäisches Patentamt

European Patent Office

Office européen des brevets

(11) Publication number: 0 393 184
A1

## EUROPEAN PATENT APPLICATION
## published in accordance with Art. 158(3) EPC

(21) Application number: 88902927.8

(22) Date of filing: 25.03.88

(86) International application number:
PCT/JP88/00309

(87) International publication number:
WO 88/07677 (06.10.88 88/22)

(51) Int. Cl.⁵: G01N 27/30, G01N 27/416

(30) Priority: 27.03.87 JP 71832/87

(43) Date of publication of application:
24.10.90 Bulletin 90/43

(84) Designated Contracting States:
BE DE FR GB IT NL SE

(71) Applicant: Terumo Kabushiki Kaisha
No. 44-1, Hatagaya 2-chome Shibuya-ku
Tokyo 151(JP)

(72) Inventor: YAMAGUCHI, Shuichiro
Terumo Kabushiki Kaisha 2656-1, Ohbuchi
Fuji-shi Shizuoka 417(JP)
Inventor: SUZUKI, Takanao
Terumo Kabushiki Kaisha 2656-1, Ohbuchi
Fuji-shi Shizuoka 417(JP)
Inventor: USHIZAWA, Norihiko
Terumo Kabushiki Kaisha 2656-1, Ohbuchi
Fuji-shi Shizuoka 417(JP)
Inventor: SHIMOMURA, Takeshi
Terumo Kabushiki Kaisha 2656-1, Ohbuchi
Fuji-shi Shizuoka 417(JP)

(74) Representative: Gillard, Marie-Louise et al
Cabinet Beau de Loménie 55, Rue
d'Amsterdam
F-75008 Paris(FR)

(54) ION SENSOR.

(57) A carbon-containing carbon layer (3) is deposited on the surface of an electrically conductive substrate to form an electrically conductive substrate, and the surface of this carbon layer is covered with ion-sensitive films (4), (5) which generate a potential corresponding to a predetermined ion concentration. The resulting ion sensor can be shaped freely, particularly, can be made very small, and can measure an ion concentration in the blood vessel or a capillary.

FIG. 1

# DESCRIPTION

## ION SENSOR

## TECHNICAL FIELD

This invention relates to an ion sensor and, more particularly, to a film-coated ion sensor fabricated using a base obtained by directly coating the surface of an electrically conductive substrate with electrically conductive carbon.

## BACKGROUND ART

A carbon material (particularly an electrically conductive carbon material) such as basal plane pyrolytic graphite (BPG) or glassy carbon is often used as the electrically conductive substrate in an ion sensor, especially a solid-type (or film-coated type) ion sensor, in order to conform to the coating film and for reasons of cost.

However, the carbon material is a base plate of a certain size in the form of a bar or disk and there are restrictions as to shape since the carbon material is limited to this plate-like configuration. For this reason, it is difficult to use the carbon material to measure ion concentration in extremely slender regions such as in capillaries and blood vessels.

## DISCLOSURE OF THE INVENTION

The present invention provides an ion sensor having any desired shape, particularly an ion sensor capable of measuring ion concentration is very slender regions such as in blood vessels and capillaries.

As means for solving the aforesaid problem, the ion sensor of the present invention comprises a carbon layer which includes carbon deposited on a surface of an electrically conductive substrate, and an ion-sensitive film coating a surface of the carbon layer for generating an electric potential corresponding to a prescribed ion concentration.

Preferably, the invention is practiced in the following manner:

The carbon layer is selected from among a carbon-containing emulsion, a carbon-containing paste, a carbon-containing toner and a carbon-containing electrically conductive binding agent.

The substrate is a fine conducting wire.

The fine conducting wire is the core of a coaxial cable.

The fine conducting wire has a diameter of 0.01 - 0.5 mm.

The substrate was made a plate of insulator or a film of insulator.

The substrate is a glass plate.

The substrate is a semiconductor plate.

The ion-sensitive film comprises a redox film having a redox function, and an ion-selective ion carrier film coating a surface of the redox film.

With such an arrangement, there is provided an ion sensor of any desired shape, which is based on a carbon substrate of any desired shape, conforming to the electrically conductive substrate. Such an ion sensor is capable of measuring ion concentration in very slender regions such as in blood vessels and capillaries.

More specifically, the ion sensor of the invention can be used for new ion measurement in the form of a needle-type sensor for clinical and animal experiments and the like in the medical field. The technique for coating an ultrafine wire with a carbon material can also be widely applied to micromachining and circuit techniques. Furthermore, it is possible to manufacture electrically conductive carbon substrates irrespective of shape. Thus, the invention contributes to expansion in the fields of ion sensor utilization and application.

BRIEF DESCRIPTION OF THE DRAWINGS

Fig. 1 is a schematic view of an ion sensor which uses a coaxial cable according to the present embodiment;

Fig. 2 is a view showing the results of measurement of a hydrogen ion sensor according to the present embodiment;

Fig. 3 is a view showing the results of measurement of a potassium ion sensor according to the present embodiment;

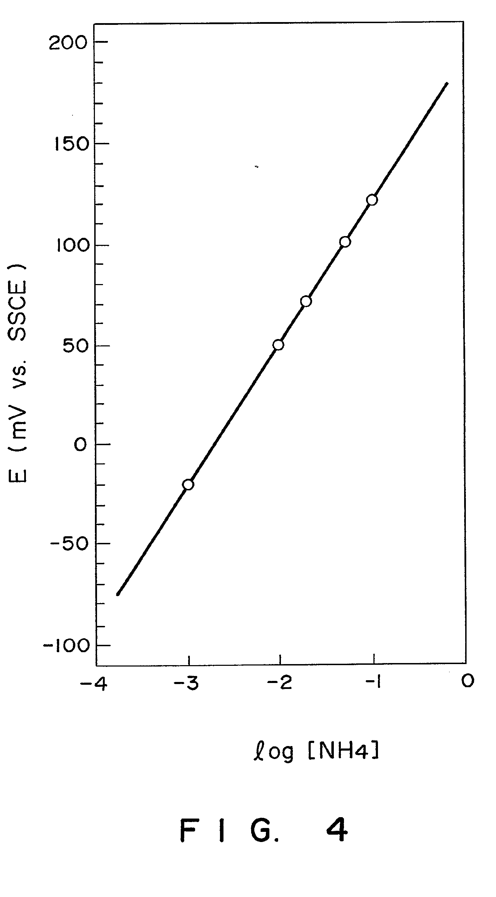

Fig. 4 is a view showing the results of measurement of an ammonium ion sensor according to the present embodiment;

Fig. 5 is a view showing the results of measurement of a calcium ion sensor according to the present embodiment; and

Fig. 6 is a view showing the results of measurement of a chlorine ion sensor according to the present embodiment.

### BEST MODE FOR CARRYING OUT THE INVENTION

<Example 1>

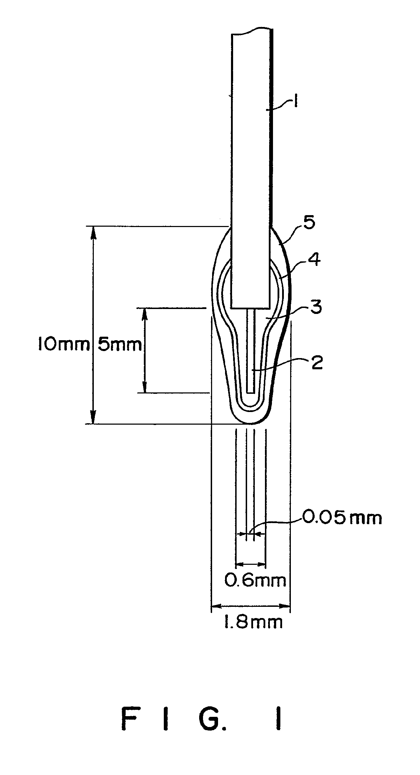

A carbon substrate was fabricated by using an ultrafine coaxial cable 1 (a four-layer coaxial cable comprising a silver wire, an insulator, a leading wire and an external insulator, in which the silver wire was a single strand having a diameter of 0.05 mm manufactured by Junkohsha Co. Ltd. under the brand name "Junflon PFA"), exposing the internal silver wire 2, as shown in Fig. 1, dip-coating a carbon paste 3 (viscosity: 150 – 300 PS, manufactured by Acheson Japan Ltd. under the brand name "JEF-010") onto a length of 5 mm of the silver wire, and thereafter performing sintering for 30 min at a sintering temperature of $150^{\circ}$C.

A hydrogen ion sensor was fabricated by depositing a redox film 4 as an ion-sensitive film on the surface of the prepared carbon substrate by an electro-oxidative reaction, the conditions whereof are given below, and then depositing a hydrogen ion carrier film 5 on the redox film 4 by a dipping process.

The electro-oxidative reaction was carried out by a three-pole system in which the carbon substrate was employed as a working electrode, an SSCE as a reference electrode and a platinum mesh as a counter electrode.

(Electro-oxidative reaction conditions)

0.5 M  2,6 - dimethylphenol

0.2 M  $NaClO_4$

in an acetonitrile solution

After the electrolytic potential was swept twice from 0V to 1.5 V (vs. a saturated sodium calomel electrode) at a scan rate of 50 mV/sec, constant potential electrolysis was performed at +1.5 V for 10 min, whereby the redox film 4 was formed.

Next, a hydrogen ion sensor 10 was fabricated by forming the hydrogen ion carrier film 5 on the surface of the redox film 4 through a dipping process (15 applications) under the following conditions:

(Hydrogen ion film composition)

TDDA (tridodecylamine)

313  mg  6  wt %

KTpClPB [potassium tetrakis(P-chlorophenyl) borate]

31.3 mg  0.6 wt %

DOS (2-ethylhexyl sebacate)

3255  mg  62.3 wt %

PVC (polyvinyl chloride)

1625  mg  31.1 wt %

THF (tetrahydrofuran) solution:  solvent <Experiment 1>

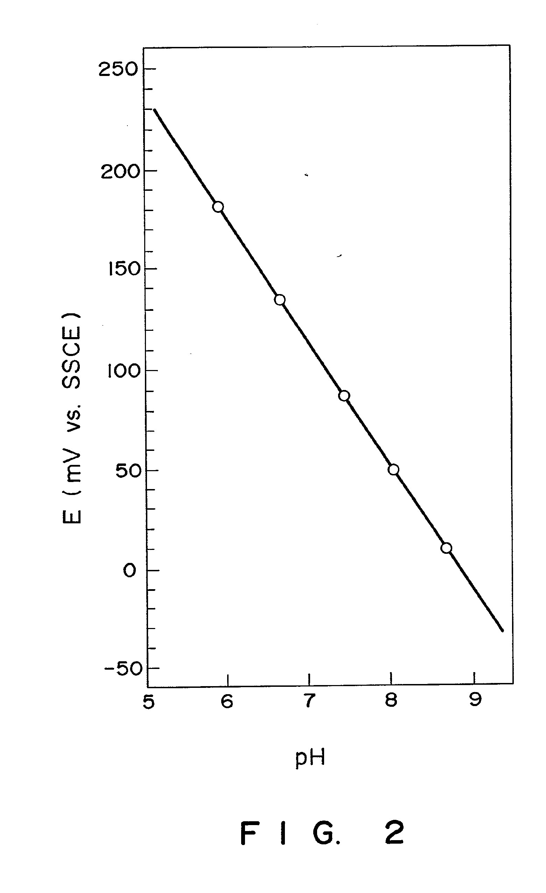

When emf response was measured over a pH range of 5 ∽ 9 in a standard phosphate buffer solution using the hydrogen ion sensor 10 was prepared as described above, the fact that a linear relationship in accordance with an ideal Nernst equation is obtained, as shown in Fig. 2, was evident from the experimental results. The slope of the straight line is 61.75 mV/pH ($37^{O}$C), which is in good agreement with the theoretical equation. The hydrogen ion sensor 10 was not influenced by the oxygen in solution over a wide range of partial pressures of oxygen ($PO_2$), i.e., 0 ∽ 760 mmHg.

<Example 2>

A carbon paste (identical with that of Example 1) was applied to a thickness of about 1.0 mm to a glass plate (length: 5 mm, width: 20 mm, thickness: 1 mm) instead of the coaxial cable 1 of Example 1. This was followed by sintering for 30 min at $150^{O}$C. One end of the carbon paste was then insulated with a silicone resin (manufactured by Toray Silicone Co. Ltd., under the brand name "PRX 305 Clear") to length and width dimensions of 5 mm x 5 mm. Enough of the other end was left to make contact with a silver wire (leading wire). The leading wire and the carbon paste were connected using a silver paste binding agent, thereby forming a carbon substrate. This carbon substrate was used to fabricate a hydrogen ion sensor just as in Example 1.

<Experiment 2>

When the correlation between the pH of a standard buffer solution (pH: 5 - 9) and the electrode emf response was measured, good agreement with the Nernst linear equation was confirmed.

<Example 3>

A silicon semiconductor substrate was used instead of the glass plate of Example 2 to fabricate a hydrogen ion sensor similar to that of Example 2.

<Experiment 3>

When the correlation between the pH of a standard buffer solution (pH: 5 - 9) and the electrode emf response was measured, good agreement with the Nernst linear equation was confirmed.

<Example 4>

A potassium ion sensor was fabricated by applying a potassium ion carrier film instead of the hydrogen ion carrier film of Example 1.

(Potassium ion carrier film composition)

| | |
|---|---|
| valinomycin | 25 mg |
| KTpClPB | 5 mg |
| DOS | 647 mg |
| PVC | 323 mg |
| THF solution: | solvent |

<Experiment 4>

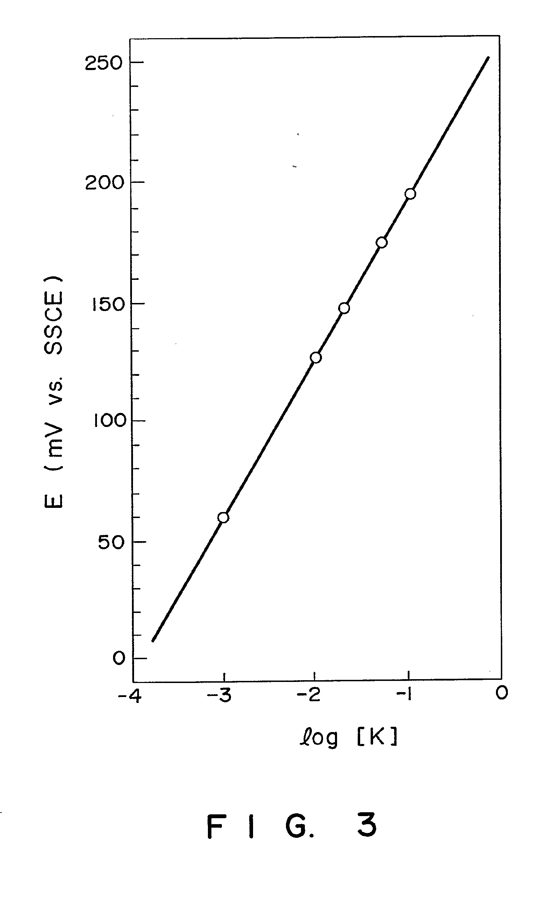

When the relationship between potassium ion concentration and emf response was found using the potassium ion sensor fabricated as described above, an excellent linear relationship was obtained, as shown in Fig. 3. The slope of this straight line is about 67.96

mV/pK ($37^{O}$C). It was found that this relationship holds over a wide range of potassium ion concentrations ($K^+$) of 1 M - $10^{-4}$ M, and that this sensor can be utilized as an excellent potassium ion sensor.

<Example 5>

An ammonium ion sensor was fabricated by applying an ammonium ion carrier film instead of the hydrogen ion carrier film of Example 1.

(Ammonium ion carrier film composition)

| nonoctyn | 25 mg |
| KTpClPB | 5 mg |
| DOS | 647 mg |
| PVC | 323 mg |

THF solution: solvent

<Experiment 5>

The relationship between ammonium ion concentration and emf response was investigated using the ammonium ion sensor fabricated as described above. A linear relationship was established over a wide range of ammonium ion concentrations ($NH_4^+$) of 1 M - $10^{-4}$ M, the slope of this straight line being about 71.2 mV/$pNH_4$ ($37^{O}$C). This sensor can be utilized as an excellent ammonium ion sensor.

<Example 6>

A calcium ion sensor was fabricated by applying a calcium ion carrier film instead of the hydrogen ion carrier film of Example 1.

(Calcium ion carrier film composition)

calcium bis[di-(n-octylphenyl) phosphate   22.5 mg

KTpClPB                                      8.6 mg

DOS                                        493.8 mg

THF solution:  solvent                     251.1 mg

<Experiment 6>

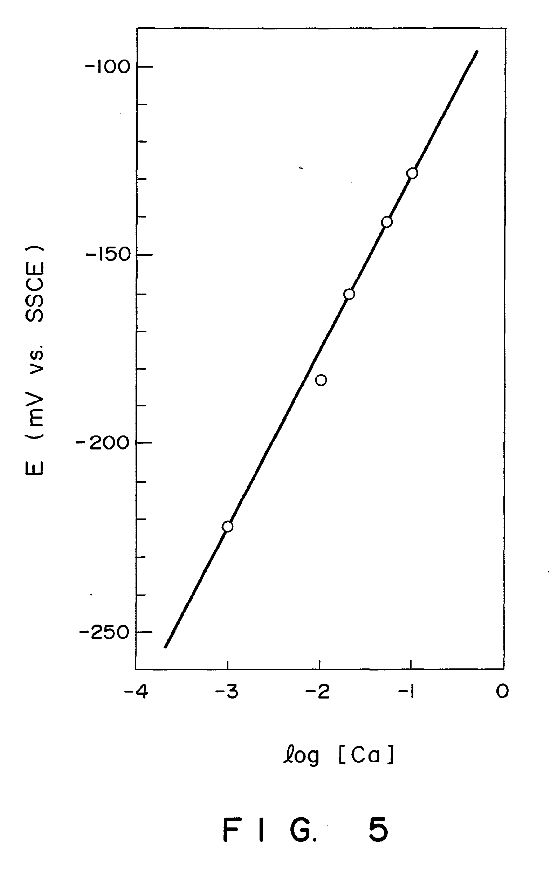

The relationship between calcium ion concentration and emf response, which was measured using the calcium ion sensor fabricated as described above, exhibits a good linear relationship, as shown in Fig. 5, the slope of the straight line being about 47.16 mV/pCa$^{2+}$ (37$^{\circ}$C) over a range of calcium ion concentrations (Ca$_2^+$) of 1 M – 10$^{-4}$ M.  Accordingly, it was found that this sensor can be utilized as an excellent calcium ion sensor.

<Example 7>

A chlorine ion sensor was fabricated by applying a chlorine ion carrier film instead of the hydrogen ion carrier film of Example 1.

(Chlorine ion carrier film composition)

triphenyl tin chloride    31.4 mg

DOS                      362.6 mg

PVC                      162.4 mg

THF solution:  solvent

<Experiment 7>

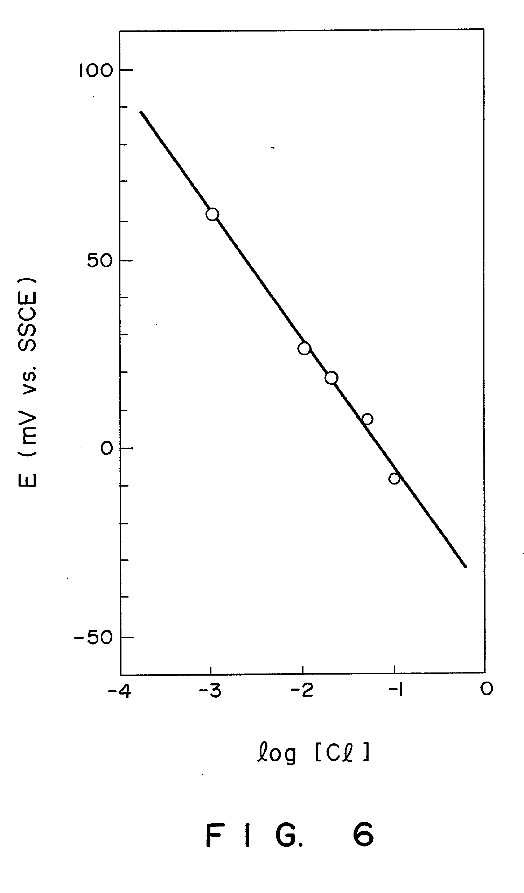

When chlorine ion concentration and emf response were measured using the chlorine ion sensor prepared as described above, an excellent linear relationship was obtained, as shown in Fig. 6.  The slope of this straight line is about 33.83 mV/pCl$^-$ over a range of chlorine ion concentrations (Cl$^-$) of 1 M $\sim$ 10$^{-4}$ M. Accordingly, this sensor can be utilized as an excellent chlorine ion sensor.

A coaxial cable, a glass plate and the like have been cited as examples of a sintered carbon substrate in the present embodiment. However, it is possible to use any chemically stable material having a prescribed strength. The present embodiment merely sites examples of the optimum ion sensor film construction and the materials thereof. Further, the invention is not limited to ion sensors for sodium ion, magnesium ion, carbon dioxide ion and acetic acid ion, and the invention naturally can be applied to other biosensors such as enzyme sensors.

CLAIMS:

1.  An ion sensor comprising:

a carbon layer which includes carbon deposited on a surface of an electrically conductive substrate; and

an ion-sensitive film coating a surface of said carbon layer for generating an electric potential corresponding to a prescribed ion concentration.

2.  The ion sensor according to claim 1, wherein said carbon layer is selected from among a carbon-containing emulsion, a carbon-containing paste, a carbon-containing toner and a carbon-containing electrically conductive bonding agent.

3.  The ion sensor according to claim 1, wherein said substrate is a fine conducting wire.

4.  The ion sensor according to claim 3, wherein said fine conducting wire is the core of a coaxial cable.

5.  The ion sensor according to claim 3, wherein said fine conductor has a diameter of 0.01 - 0.5 mm.

6.  The ion sensor according to claim 1, wherein said substrate is a glass plate.

7.  The ion sensor according to claim 1, wherein said substrate is a semiconductor plate.

8.  The ion sensor according to claim 1, wherein said ion-sensitive film comprises a redox film having a redox function, and an ion-selective ion carrier film coating a surface of said redox film.

F I G. I

F I G. 2

F I G. 3

F I G.   4

F I G. 5

FIG. 6

# INTERNATIONAL SEARCH REPORT

International Application No PCT/JP88/00309

## I. CLASSIFICATION OF SUBJECT MATTER (if several classification symbols apply, indicate all) *

According to International Patent Classification (IPC) or to both National Classification and IPC

Int.Cl⁴    G01N27/30, 27/46

## II. FIELDS SEARCHED

Minimum Documentation Searched [7]

| Classification System | Classification Symbols |
|---|---|
| IPC | G01N27/30, 27/46-27/56 |

Documentation Searched other than Minimum Documentation
to the Extent that such Documents are Included in the Fields Searched [8]

Jitsuyo Shinan Koho      1926 - 1988
Kokai Jitsuyo Shinan Koho    1971 - 1988

## III. DOCUMENTS CONSIDERED TO BE RELEVANT [9]

| Category * | Citation of Document, [11] with indication, where appropriate, of the relevant passages [12] | Relevant to Claim No. [13] |
|---|---|---|
| Y | JP, A, 62-21054 (Terumo Corporation) 29 January 1987 (29. 01. 87) (Family: none) | 1-8 |
| Y | JP, A, 62-21055 (Terumo Corporation) 29 January 1987 (29. 01. 87) (Family: none) | 1-8 |
| Y | JP, A, 58-172541 (Terumo Corporation) 11 October 1983 (11. 10. 83) (Family: none) | 1-8 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| June 13, 1988 (13. 06. 88) | June 27, 1988 (27. 06. 88) |

| International Searching Authority | Signature of Authorized Officer |
|---|---|
| Japanese Patent Office | |

Form PCT ISA 210 (second sheet) (January 1985)